# EUROPEAN PATENT SPECIFICATION

(11) **EP 3 579 248 B1**
(45) Date of publication and mention of the grant of the patent: **13.09.2023**
(21) Application number: 18748275.7
(22) Date of filing: 01.02.2018
(51) Int. Cl.: G21K 5/04, A61L 2/08, B65B 55/08, G21K 1/093, G21K 5/10, B65B 55/04

(54) **NOZZLE-TYPE ELECTRON BEAM IRRADIATION DEVICE**
ELEKTRONENBESTRAHLUNGSVORRICHTUNG VOM DÜSENTYP
DISPOSITIF D'IRRADIATION PAR FAISCEAU D'ÉLECTRONS DE TYPE À BUSE

(30) Priority: 03.02.2017 JP 2017018020
(43) Date of publication of application: 11.12.2019
(73) Proprietor: Hitachi Zosen Corporation, Osaka-shi, Osaka 559-8559 (JP)
(72) Inventor: NODA, Takeshi, Osaka-shi Osaka 559-8559 (JP); TANAKA, Ryuta, Osaka-shi Osaka 559-8559 (JP); SAKAI, Ichiro, Osaka-shi Osaka 559-8559 (JP)
(74) Representative: Canzler & Bergmeier Patentanwälte Partnerschaft mbB
(86) International application number: PCT/JP2018/003339
(87) International publication number: WO 2018/143319

(56) References cited:
- WO-A1-2012/057166
- WO-A1-2014/175065
- WO-A1-2014/175065
- JP-A- 2002 104 333
- JP-A- 2005 331 418
- JP-A- 2012 055 556
- JP-A- 2013 129 453
- JP-B1- 6 068 693
- JP-B1- 6 068 693
- US-A- 5 254 856
- US-A- 5 313 061
- JOHN F O'HANLON ED - JOHN F O'HANLON: "A User's Guide to Vacuum Technology, Getter and Ion Pumps, High Vacuum Systems", 1 January 2003 (2003-01-01), A USER'S GUIDE TO VACUUM TECHNOLOGY, JOHN WILEY & SONS, INC, HOBOKEN, NJ, USA, PAGE(S) 247 - 262, 379, XP002537656, ISBN: 978-0-471-27052-2 * page 248; figure 14.1 *

## Description

The present invention relates to a nozzle-type electron beam irradiation device.

A nozzle-type electron beam irradiation device includes a vacuum nozzle that is connected to a vacuum chamber and emits an electron beam from the tip of the nozzle. With this configuration, the vacuum nozzle of the nozzle-type electron beam irradiation device is inserted into the opening of a container and an electron beam is emitted from the vacuum nozzle, thereby irradiating the inside of the container with the electron beam. An electron beam is emitted into the container, for example, to sterilize the inside of the container. In this case, the nozzle-type electron beam irradiation device sterilizes the inside of the container by direct irradiation of an electron beam, thereby suppressing deterioration of the container as well as power consumption as compared with an electron beam irradiation device that emits a strong electron beam into a container from the outside.

In such a nozzle-type electron beam irradiation device, it is preferable that an electron beam guided into a vacuum nozzle hardly hits the inner surface of the vacuum nozzle. This is because an electron beam hitting the inner surface of the vacuum nozzle may not be emitted to the outside and cause a loss that reduces the yield of the electron beam. A nozzle-type electron beam irradiation device disclosed in the related art focuses an electron beam with an electrostatic lens provided in an electron beam generator (for example, JP 5597743 B2).
US 5 254 856 A relates to improvements in the electron microscope, electron beam lithography apparatus, ion microscope, secondary ion mass spectrometer or other charged particle beam apparatus employing a finely focused charged particle beam (electron or ion beam). Still more particularly, US 5 254 856 A relates to structural improvements in a charged particle beam apparatus that uses an electrostatic lens for finely focusing the charged particle beam. According to this known apparatus a nozzle-type electron beam irradiation device comprising a vacuum nozzle insertable into an opening of an irradiation object, the opening being formed on the irradiation object, the nozzle-type electron beam irradiation device comprising a vacuum chamber and an electron beam generator disposed in the vacuum chamber. The vacuum nozzle is connected to the vacuum chamber so as to guide an electron beam from the electron beam generator and emit the electron beam to outside. The nozzle-type electron beam irradiation device comprising a magnet that surrounds the vacuum nozzle from the outside and focuses the electron beam guided into the vacuum nozzle and an inserting/removing mechanism that inserts and removes the vacuum nozzle into and from the opening of the irradiation object.
According to US 5 313 061 A a portable analytical grade mass spectrometer system contained in a single enclosure is disclosed for use in analyzing atmospheric, water, soil, drugs, explosives and other substances and includes a gas chromatograph and a mass analyzer assembly enclosed within a vacuum housing, a vacuum pump, and an on-board computer such that an operator, by means of an attached keyboard, can input data and information, and input a sample to be analyzed, and thereby operate the miniaturized mass spectrometer system. In the design example of this system it is preferred that the appendage ion pump magnets and the 90° analyzer magnets are made of neodymium-boron-iron.
Regarding cooling it is known from JOHN F O'HANLON ED - JOHN F O'HANLON: "A User's Guide to Vacuum Technology, Getter and Ion Pumps, Hight Vacuum Systems" that active gases are captured on the fresh titanium surface, which is cooled with water or liquid nitrogen.
JP 6 068693 B1 shows and describes an electron beam irradiation device including an electron gun for generating electron beams, a vacuum chamber for composing a vacuum space for storing the electron gun; a guide part, where a base end side is connected to the vacuum chamber to communicate with the vacuum space and a long cylindrical member insertable into a bottle via an opening part of the bottle is provided at a tip side, and the electron beams generated by the electron gun pass inside; an electron beam emission window that is provided at a tip side of the guide part and emits the electron beams; and an adjustment electromagnetic coil for adjusting a track of the electron beams in the guide part. The adjustment electromagnetic coil is disposed at the base end of the guide part outside the vacuum space.
From WO 2014/175065 A1 it is an electron beam irradiation apparatus known that emits an electron beam into a container, the electron beam irradiation apparatus including: a vacuum housing constituting a vacuum chamber; an electron generator provided in the vacuum housing; a cylindrical nozzle member that is extended from the vacuum housing so as to be inserted into the container and has exit windows on the distal end of the nozzle member, the exit windows being provided for emission of an electron beam generated by the electron generator into the container; and a magnetic shield member for the vacuum chamber and a magnetic shield member for the nozzle member, the magnetic shield members being respectively provided for the vacuum housing and the nozzle member so as to block variable magnetism generated around an electron beam trajectory extended from the electron generator to the exit windows.

In the nozzle-type electron beam irradiation device described in JP 5597743 B2, although an electron beam is focused through the electrostatic lens, the focusing may be insufficient in some cases. For example, in the case of a long vacuum nozzle, an electron beam may widely hit the inner surface of the vacuum nozzle until the electron beam reaches the tip of the vacuum nozzle, so that the yield of the electron beam may decrease. Thus, if an electron beam is generated with low power by the electron beam generator, the electron beam may not be sufficiently emitted from the vacuum nozzle. For this reason, it is necessary to generate an electron beam with high power in the electron beam generator, thereby shortening the life of a cathode in the electron beam generator. If an electron beam focusing mechanism is additionally provided, an electron beam is sufficiently focused but the configuration becomes complicated. Furthermore, the electron beam focusing mechanism may interrupt the insertion of the vacuum nozzle into the opening of a container, preventing the inside of the container (an irradiation object having an opening) from being properly irradiated with an electron beam.

An object of the present invention is to provide a nozzle-type electron beam irradiation device that can achieve a long life with a simple configuration and properly emit an electron beam into an irradiation object having an opening.

In order to solve the problems, a nozzle-type electron beam irradiation device according to the invention includes a vacuum nozzle insertable into the opening of an irradiation object, the opening being formed on the irradiation object, the irradiation device emitting an electron beam into the irradiation object through the vacuum nozzle,
the nozzle-type electron beam irradiation device including a vacuum chamber and an electron beam generator disposed in the vacuum chamber,
the vacuum nozzle being connected to the vacuum chamber so as to guide an electron beam from the electron beam generator and emit the electron beam to the outside,
the nozzle-type electron beam irradiation device including:
   a magnet that surrounds the vacuum nozzle from the outside and focuses the electron beam guided into the vacuum nozzle;
   an inserting/removing mechanism that inserts and removes the vacuum nozzle into and from the opening of the irradiation object;
   whereby the magnet is a neodymium magnet and the irradiation device further comprising:
      a cooling mechanism that cools the vacuum nozzle,
      the cooling mechanism cooling the neodymium magnet so as to prevent irreversible demagnetization while cooling the vacuum nozzle,
      a connecting member connecting the vacuum chamber and the vacuum nozzle,
      the connecting member containing the neodymium magnet inside the connecting member,
      the vacuum nozzle is disposed with a base end embedded in the connecting member, and
      the cooling mechanism includes a coolant passage that allows the passage of a coolant,
      the coolant passage being disposed between the vacuum nozzle and the neodymium magnet so as to surround the vacuum nozzle in the connecting member.

### Advantageous Effects of Invention

The nozzle-type electron beam irradiation device is applicable even if an electron beam is generated with low power, achieving a long life. Furthermore, another cooling mechanism for cooling the neodymium magnet is not necessary, achieving a simple configuration. Additionally, the electron beam is emitted into the irradiation object while the vacuum nozzle is inserted and removed into and from the opening of the irradiation object without being interrupted by the neodymium magnet. This can properly emit an electron beam into the irradiation object.

### Brief Description of Drawings

[FIG. 1] FIG. 1 is a schematic longitudinal section illustrating a nozzle-type electron beam irradiation device not having all the features of the present claim and thus not according to an embodiment of the present invention.
[FIG. 2] FIG. 2 is a schematic longitudinal section illustrating the nozzle-type electron beam irradiation device according to Example 1 of the present invention.
[FIG. 3] FIG. 3 is an exploded perspective view of the nozzle-type electron beam irradiation device viewed from a PET bottle.
[FIG. 4] FIG. 4 is a cross-sectional view taken along line A-A of FIG. 2.
[FIG. 5] FIG. 5 is a cross-sectional view taken along line B-B of FIG. 2.
[FIG. 6] FIG. 6 is a schematic longitudinal section illustrating the nozzle-type electron beam irradiation device according to Example 2 of the present invention. Description of Embodiment

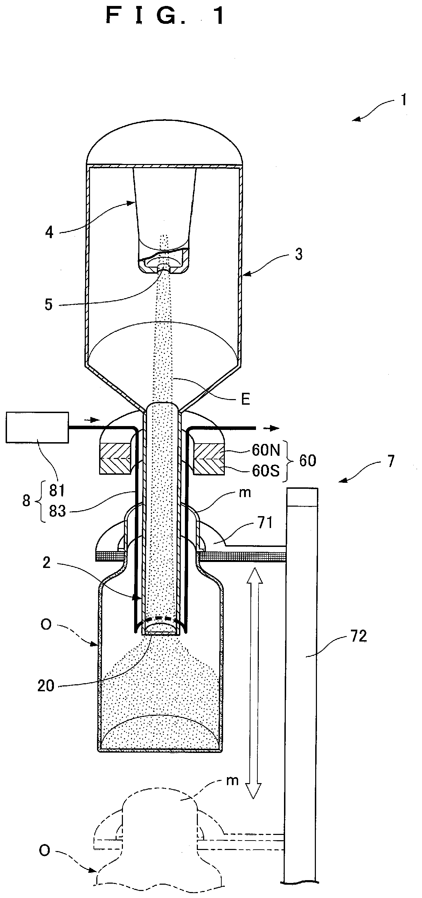

Referring to FIG. 1, a nozzle-type electron beam irradiation device 1 useful for understanding the present invention will be described in detail below.

As illustrated in FIG. 1, the nozzle-type electron beam irradiation device 1 includes a vacuum nozzle 2 insertable into an opening m of an irradiation object O, the opening m being formed on the irradiation object O. An electron beam E is emitted into the irradiation object O through the vacuum nozzle 2. In this case, the irradiation object O having the opening m is, for example, a container such as a PET bottle or a preform. The preform is a material form shaped like a test tube before being molded into a PET bottle by blow molding. For simplicity, the irradiation object O having the opening m will be discussed as a PET bottle O.

The nozzle-type electron beam irradiation device 1 includes a vacuum chamber 3 and an electron beam generator 4 disposed in the vacuum chamber 3. The electron beam generator 4 may be provided with an electrostatic lens 5 that focuses the electron beam E from the electron beam generator 4. The vacuum nozzle 2 is connected to the vacuum chamber 3 so as to guide the electron beam E from the electron beam generator 4 and emit the electron beam E to the outside.

The inside of the vacuum chamber 3 is evacuated so as to accelerate the electron beam E from the electron beam generator 4. The electron beam generator 4 includes, for example, a component like a cathode for generating the electron beam E. The vacuum nozzle 2 has an exit window 20 made of, for example, titanium foil at the tip of the nozzle in order to emit the electron beam E to the outside. If the electrostatic lens 5 is provided in the electron beam generator 4, the electrostatic lens 5 is disposed so as to guide the electron beam E from the electron beam generator 4 into the vacuum nozzle 2.

In this case, the degree of focusing of the electron beam E greatly changes according to the position of a component (e.g., a cathode) for generating the electron beam E, for example, a distance between the cathode and the electrostatic lens 5. Even if the position (distance) is properly set, in the case of the long vacuum nozzle 2, the electron beam E hits the inner surface of the vacuum nozzle 2 before reaching the tip of the vacuum nozzle 2. Hence, as a gist of the present invention, a neodymium magnet 60 for focusing the electron beam E guided by the vacuum nozzle 2 is disposed outside the vacuum nozzle 2 so as not to interfere with the electron beam E. Generally, a neodymium magnet has a stronger magnetic force than other permanent magnets and thus can be smaller in size than other permanent magnets.

The neodymium magnet 60 is shaped so as to surround the vacuum nozzle 2 from the outside (FIG. 1 illustrates only a half of the magnet to enhance ease of seeing). The neodymium magnet 60 is located at a place where the neodymium magnet 60 does not interrupt the insertion of the vacuum nozzle 2 into the opening m of the PET bottle O, for example, a base end of the vacuum nozzle 2. Moreover, the neodymium magnet 60 is disposed with a vacuum-chamber side 60N serving as the north pole (or the south pole) and a PET-bottle side 60S serving as the south pole (or the north pole).

The nozzle-type electron beam irradiation device 1 further includes an inserting/removing mechanism 7 that inserts and removes the vacuum nozzle 2 into and from the opening m of the PET bottle O, and a cooling mechanism 8 that cools the vacuum nozzle 2.

The inserting/removing mechanism 7 may be, for example, a PET bottle elevator 7 that moves up and down the PET bottle O, but is not particularly limited as long as the vacuum nozzle 2 is inserted and removed into and from the opening m of the PET bottle O. The PET bottle elevator 7 lifts the PET bottle O so as to insert the tip (exit window 20) of the vacuum nozzle 2 into the PET bottle O from the opening m and then moves down the PET bottle O from the state so as to remove the tip of the vacuum nozzle 2 from the opening m of the PET bottle O. For this operation, the PET bottle elevator 7 includes a gripper 71 that grips the PET bottle O, a guide member 72 that vertically guides the gripper 71, and an actuator (not shown) that vertically moves the gripper 71 according to the guide of the guide member 72. The inserting/removing mechanism 7 may move up and down the vacuum nozzle 2 unlike the PET bottle elevator 7 that moves up and down the PET bottle O.

The cooling mechanism 8 cools the vacuum nozzle 2, which rises in temperature while guiding the electron beam E, to a proper temperature. Hence, the cooling mechanism 8 is provided with a coolant passage 83 surrounding the vacuum nozzle 2 and a coolant introducing portion 81 that allows the passage of a coolant through the coolant passage 83. The coolant passage 83 allows the passage of one of a liquid and gaseous coolant therein, so that the coolant absorbs heat from the vacuum nozzle 2. In this configuration, the neodymium magnet 60 advantageously has a stronger magnetic force than other permanent magnets but disadvantageously tends to be irreversibly demagnetized by heat. Thus, the cooling mechanism 8 cools the neodymium magnet 60 so as to prevent irreversible demagnetization while cooling the vacuum nozzle 2. This eliminates the need for another cooling mechanism for cooling the neodymium magnet 60.

With this configuration, the electron beam E from the electron beam generator 4 disposed in the vacuum chamber 3 is guided into the vacuum nozzle 2 and then is emitted from the exit window 20 of the vacuum nozzle 2 to the outside. If the electrostatic lens 5 is provided, the electron beam E is focused through the electrostatic lens 5 before being guided into the vacuum nozzle 2. The electron beam E guided into the vacuum nozzle 2 is focused by the neodymium magnet 60 surrounding the vacuum nozzle 2 from the outside. This reduces the dose of the electron beam E hitting the inner surface of the vacuum nozzle 2, leading to a smaller loss of the electron beam E emitted to the outside. Thus, the yield of the electron beam E increases, achieving applicability even if the electron beam E is generated with low power by the electron beam generator 4.

While the PET bottle elevator 7 inserts and/or removes the vacuum nozzle 2 into and/or from the opening m of the PET bottle O, the electron beam E is emitted into the PET bottle O. In this case, the neodymium magnet 60 that can be small in size does not interrupt the insertion and removal.

In this way, the nozzle-type electron beam irradiation device 1 is applicable even if the electron beam E is generated with low power, achieving a long life. Furthermore, another cooling mechanism for cooling the neodymium magnet 60 is not necessary, achieving a simple configuration. Additionally, the electron beam E is emitted into the PET bottle O while the vacuum nozzle 2 is inserted and removed into and from the opening m of the PET bottle O without being interrupted by the neodymium magnet 60. This can properly emit the electron beam E into the PET bottle O.

The nozzle-type electron beam irradiation device 1 according to the embodiment will be more specifically described according to Examples 1 and 2. In Examples 1 and 2, the neodymium magnet 60, a configuration near the neodymium magnet 60, and the cooling mechanism 8 will be more specifically described. Other configurations are identical to those of the embodiment and thus the explanation thereof is omitted.

### Example 1

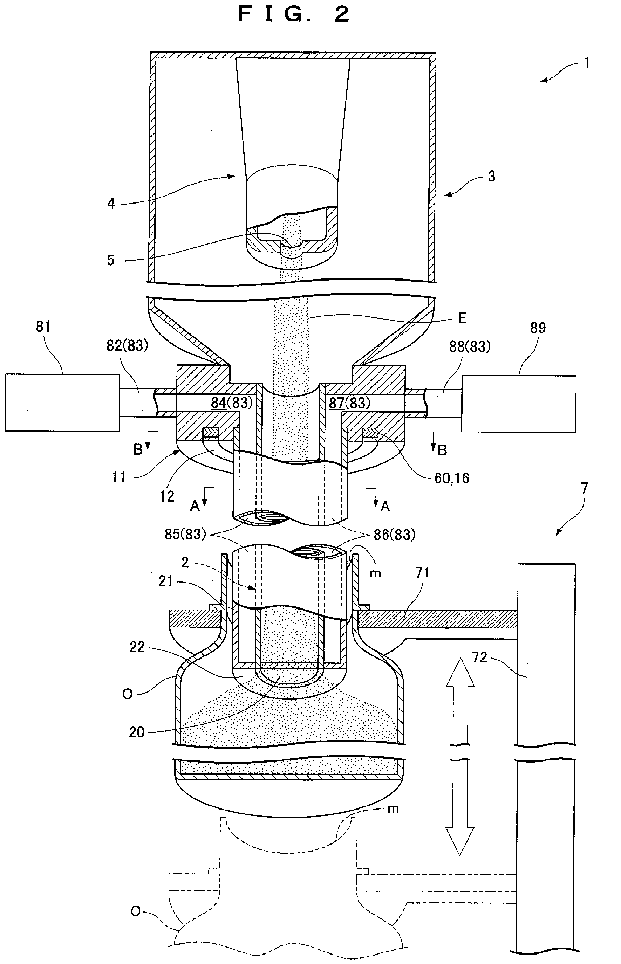

Referring to FIGS. 2 to 5, the nozzle-type electron beam irradiation device 1 according to Example 1 will be described in detail below.

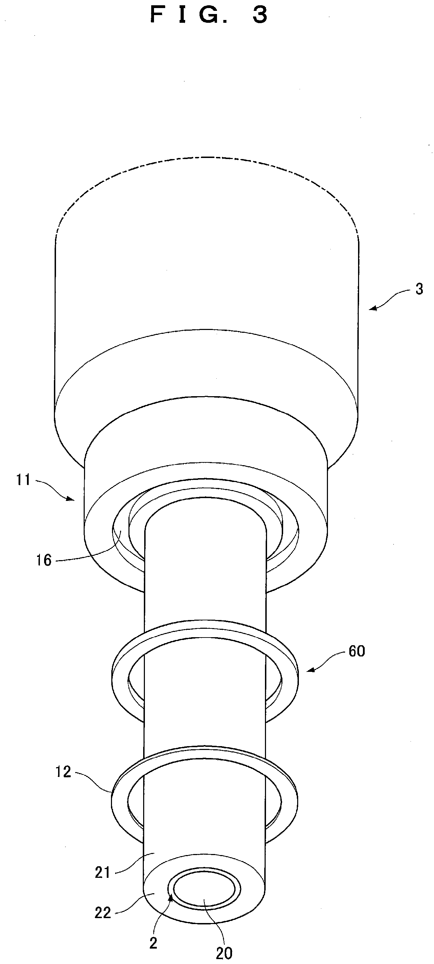

As illustrated in FIG. 2, the nozzle-type electron beam irradiation device 1 according to Example 1 includes a connecting member 11 that is a circular mount connecting the vacuum chamber 3 and the vacuum nozzle 2 (FIG. 2 illustrates only a half of the connecting member to enhance ease of seeing). The connecting member 11 fixes the vacuum chamber 3 on one side and fixes the embedded base end of the vacuum nozzle 2 on the other side, thereby structurally stabilizing the vacuum chamber 3 and the vacuum nozzle 2. Furthermore, a storage groove 16 that stores the neodymium magnet 60 is formed from the other side of the connecting member 11. The connecting member 11 further includes a lid plate 12 for fixing the neodymium magnet 60 stored in the storage groove 16 (that is, held in the storage groove 16). As illustrated in FIG. 3, the storage groove 16 is identical to the neodymium magnet 60 in plan view and has a depth equivalent to the thickness of the neodymium magnet 60 and the lid plate 12. The lid plate 12 is identical to the neodymium magnet 60 and the storage groove 16 in plan view. The neodymium magnet 60 need not be a single magnet as illustrated in FIG. 3, and may include multiple neodymium magnets in the thickness direction. Thus, a magnetic force for focusing the electron beam E can be adjusted by increasing or reducing the number of neodymium magnets 60 stored in the storage groove 16.

The cooling mechanism 8 of the nozzle-type electron beam irradiation device 1 according to Example 1 is a water-cooling mechanism that includes, as illustrated in FIG. 2, the coolant passage 83 surrounding the vacuum nozzle 2, the coolant introducing portion 81 that allows the introduction of a coolant (water) into the coolant passage 83, and a coolant collecting portion 89 from which the coolant introduced into the coolant passage 83 is collected.

The coolant passage 83 includes, as a portion for introducing a coolant to the outer edge of the tip of the vacuum nozzle 2, an introducing tube 82 that introduces the coolant from the coolant introducing portion 81 into the connecting member 11, an internal introducing passage 84 that introduces the coolant into one semicircular portion on the outer periphery of the base end of the vacuum nozzle 2 in the connecting member 11, and an external introducing passage 85 that introduces the coolant from the internal introducing passage 84 to the outer edge of the tip of the vacuum nozzle 2 (one semicircular portion) outside the connecting member 11. The coolant passage 83 includes, as a portion for collecting a coolant from the outer edge of the tip of the vacuum nozzle 2, an external collecting passage 86 that collects the coolant from the outer edge (the other semicircular portion) of the tip of the vacuum nozzle 2 to the inside of the connecting member 11, an internal collecting passage 87 that collects the coolant from the other semicircular portion on the outer periphery of the base end of the vacuum nozzle 2 in the connecting member 11, and a collecting tube 88 that collects the coolant from the inside of the connecting member 11 to the coolant collecting portion 89.

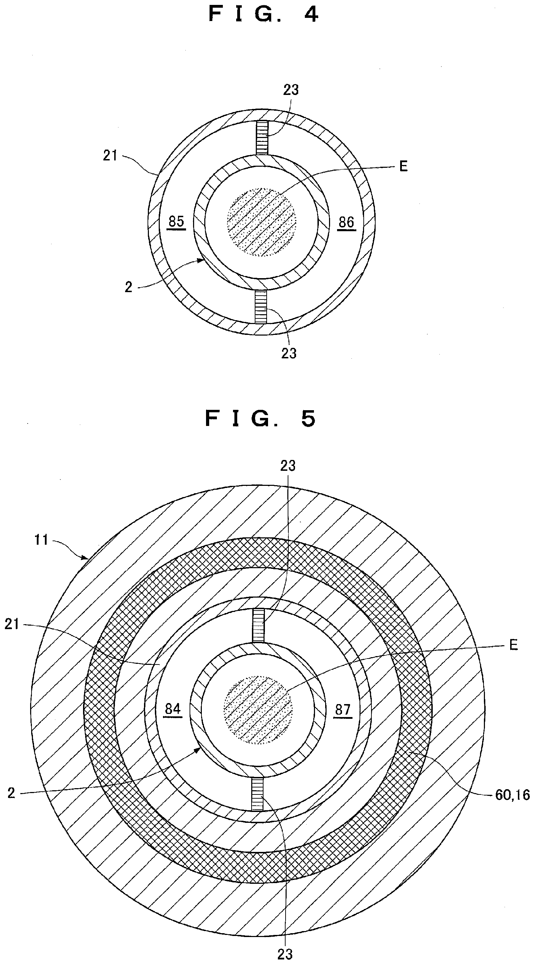

In order to form the external introducing passage 85 and the external collecting passage 86, the cooling mechanism 8 has an external cylinder 21 that is disposed so as to leave a clearance from the vacuum nozzle 2 on the outer periphery of the vacuum nozzle 2. Moreover, in order to prevent a coolant introduced into the outer edge of the tip of the vacuum nozzle 2 from flowing out of the vacuum nozzle 2, the cooling mechanism 8 further includes a tip member 22 connecting the tip end of the external cylinder 21 and the tip of the vacuum nozzle 2. As illustrated in FIG. 4 (a cross-sectional view taken along line A-A of FIG. 2), the clearance between the vacuum nozzle 2 and the external cylinder 21 corresponds to the external introducing passage 85 and the external collecting passage 86, and two partitions 23 separating the external introducing passage 85 and the external collecting passage 86 except for the outer edge of the tip of the vacuum nozzle 2 are disposed in the clearance. As illustrated in FIG. 5 (a cross-sectional view taken along line B-B of FIG. 2), also in the connecting member 11, the partitions 23 are extended so as to separate one semicircular portion and the other semicircular portion on the outer periphery of the base end of the vacuum nozzle 2. As is evident from FIG. 5, in the connecting member 11, the coolant passage 83 (specifically, portions separated by the partitions 23 in the internal introducing passage 84 and the internal collecting passage 87) is disposed between the vacuum nozzle 2 and the neodymium magnet 60 so as to surround the vacuum nozzle 2.

With this configuration, as illustrated in FIG. 2, the connecting member 11 structurally stabilizes the vacuum chamber 3 and the vacuum nozzle 2. This prevents a change of the relative positions of the vacuum chamber 3 and the vacuum nozzle 2 even on a movable member, e.g., a turntable. Moreover, the neodymium magnet 60 is held in the connecting member 11 and thus is located near the electron beam generator 4 without interrupting the insertion and removal of the vacuum nozzle 2 into and from the opening m of the PET bottle O. Furthermore, the coolant passage 83 (specifically, the internal introducing passage 84, the external introducing passage 85, the external collecting passage 86, and the internal collecting passage 87) substantially cools the overall vacuum nozzle 2 from the outer periphery (see FIGS. 4 and 5) and substantially cools the overall neodymium magnet 60 from the inner periphery (see FIG. 5).

As has been discussed, the nozzle-type electron beam irradiation device 1 according to Example 1 does not change the relative positions of the vacuum chamber 3 and the vacuum nozzle 2 in addition to the effect of the embodiment, thereby properly emitting the electron beam E into the PET bottle O.

Moreover, the neodymium magnet 60 is located near the electron beam generator 4, promoting the focusing of the electron beam E. This achieves applicability even if the electron beam E is generated with lower power, thereby further extending the life of the device. Furthermore, the electron beam E is emitted into the PET bottle O while the vacuum nozzle 2 is inserted and removed into and from the opening m of the PET bottle O without being interrupted by the neodymium magnet 60. This can more properly emit the electron beam E into the PET bottle O.

Additionally, the coolant passage 83 substantially entirely cools the vacuum nozzle 2 and the neodymium magnet 60. This efficiently cools the vacuum nozzle 2 and the neodymium magnet 60, thereby further extending the life of the overall device.

### Example 2

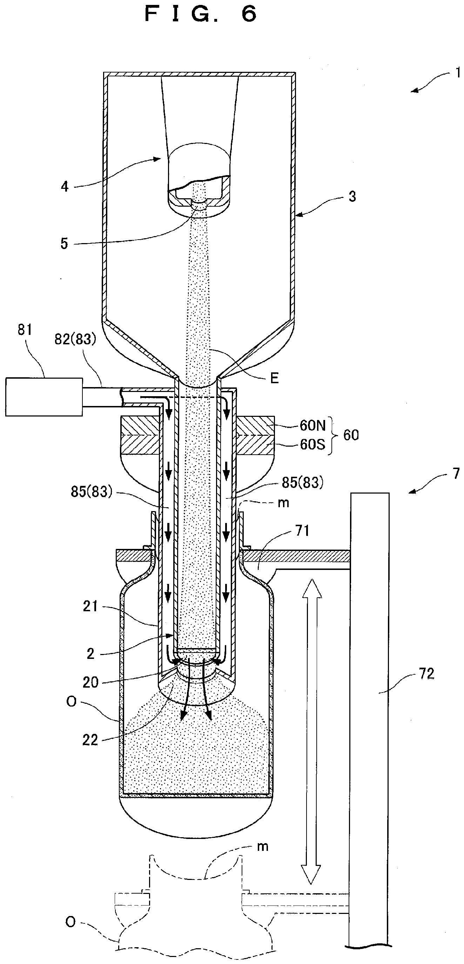

Referring to FIG. 6, the nozzle-type electron beam irradiation device 1 according to Example 2 will be described in detail below.

In Example 1, the cooling mechanism 8 is a water-cooling mechanism, whereas in Example 2, the cooling mechanism 8 is an air-cooling mechanism. Hereinafter, differences from Example 1 will be mainly described. The same configurations as those of Example 1 are indicated by the same reference numerals and the explanation thereof is omitted.

In the nozzle-type electron beam irradiation device 1 according to Example 1, the external introducing passage 85 where a coolant (water) is introduced from the base end to the tip end and the external collecting passage 86 where the coolant is collected from the tip end to the base end are divided by the partitions 23 on the outer periphery of the vacuum nozzle 2 (between the vacuum nozzle 2 and the external cylinder 21). However, the nozzle-type electron beam irradiation device 1 according to Example 2 is not provided with the partitions 23. As illustrated in FIG. 6, a coolant (nitrogen gas indicated by black arrows in FIG. 6) is introduced into the outer edge of the tip of the vacuum nozzle 2, is introduced onto the exit window 20 at the tip of the nozzle so as to impinge on the exit window 20, and then is emitted to the outside. Thus, in the cooling mechanism 8 of the nozzle-type electron beam irradiation device 1 according to Example 2, the external cylinder 21 is extended so as to project from the tip of the vacuum nozzle 2 and the tip member 22 is inclined to the exit window 20 leaving a clearance from the vacuum nozzle 2. Thus, in the coolant passage 83 of the nozzle-type electron beam irradiation device 1 according to Example 2, a part where a coolant is introduced from the outer periphery of the base end of the vacuum nozzle 2 and impinges on the exit window 20 corresponds to the external introducing passage 85. Since a coolant is discharged out of the nozzle-type electron beam irradiation device 1 according to Example 2, the configurations 86 to 89 for collecting the coolant according to Example 1 are not necessary.

As has been discussed, the overall configuration of the nozzle-type electron beam irradiation device 1 according to Example 2 can be further simplified in addition to the effect of the embodiment because the cooling mechanism 8 is an air-cooling mechanism.

The coolant passage 83 (specifically, the external introducing passage 85) substantially entirely cools the vacuum nozzle 2 and the neodymium magnet 60. This efficiently cools the vacuum nozzle 2 and the neodymium magnet 60, thereby further extending the life of the overall device.

Furthermore, the connecting member 11 according to Example 1 is used for the nozzle-type electron beam irradiation device 1 according to Example 2.

The shape of the neodymium magnet 60 is circular (annular) in the embodiment and Examples 1 and 2 but is not limited thereto. The neodymium magnet 60 may have any shape surrounding the vacuum nozzle 2, e.g., a square shape.

The coolant is water in Example 1 but is not limited thereto. Any liquid is applicable as long as heat is absorbed and transferred from the vacuum nozzle 2 and the neodymium magnet 60. Likewise, the coolant is nitrogen gas in Example 2 but is not limited thereto. Any gas is applicable as long as heat is absorbed and transferred from the vacuum nozzle 2 and the neodymium magnet 60.

## Claims

1. nozzle-type electron beam irradiation device (1) comprising a vacuum nozzle (2) insertable into an opening (m) of an irradiation object (O), the opening (m) being formed on the irradiation object (0),
the nozzle-type electron beam irradiation device (1) comprising a vacuum chamber (3) and an electron beam generator (4) disposed in the vacuum chamber (3),
the vacuum nozzle (2) being connected to the vacuum chamber (3) so as to guide an electron beam (E) from the electron beam generator (4) and emit the electron beam (E) to outside,
the nozzle-type electron beam irradiation device (1) comprising:
a magnet that surrounds the vacuum nozzle (2) from the outside and focuses the electron beam (E) guided into the vacuum nozzle (2);
an inserting/removing mechanism (7) that inserts and removes the vacuum nozzle (2) into and from the opening (m) of the irradiation object (O);
a connecting member (11) connecting the vacuum chamber (3) and the vacuum nozzle (2),
the connecting member (11) containing the magnet (60) inside the connecting member (11),
the vacuum nozzle (2) being disposed with a base end
embedded in the connecting member (11); **characterized in that** the magnet is a neodymium magnet (60), and **in that** the nozzle-type electron beam irradiation device further comprises a cooling mechanism (8) that cools the vacuum nozzle (2), the cooling mechanism (8) cooling the neodymium magnet (60) so as to prevent irreversible demagnetization while cooling the vacuum nozzle (2),
the cooling mechanism (8) comprising a coolant passage (83) that allows passage of a coolant,
the coolant passage (83) being disposed between the vacuum nozzle (2) and the neodymium magnet (60) so as to surround the vacuum nozzle (2) in the connecting member (11) .

## Patentansprüche

1. Elektronenstrahl-Bestrahlungsvorrichtung vom Düsentyp (1), umfassend eine Vakuumdüse (2), die in eine Öffnung (m) eines Bestrahlungsobjekts (O) einsetzbar ist, wobei die Öffnung (m) auf dem Bestrahlungsobjekt (O) ausgebildet ist,
wobei die Elektronenstrahl-Bestrahlungsvorrichtung vom Düsentyp (1) eine Vakuumkammer (3) und einen Elektronenstrahlgenerator (4) umfasst, der in der Vakuumkammer (3) angeordnet ist,
wobei die Vakuumdüse (2) mit der Vakuumkammer (3) so verbunden ist, dass sie einen Elektronenstrahl (E) vom Elektronenstrahlgenerator (4) leitet und den Elektronenstrahl (E) nach außen emittiert,
wobei die Elektronenstrahl-Bestrahlungsvorrichtung vom Düsentyp (1) umfasst:
einen Magneten, der die Vakuumdüse (2) von außen umgibt und den in die Vakuumdüse (2) geleiteten Elektronenstrahl (E) fokussiert;
eine Einführungs-/Entnahmevorrichtung (7), die die Vakuumdüse (2) in die Öffnung (m) des Bestrahlungsobjekts (O) einführt und aus ihr entnimmt;
ein Verbindungselement (11), das die Vakuumkammer (3) und die Vakuumdüse (2) verbindet,
wobei das Verbindungselement (11) den Magneten (60) innerhalb des Verbindungselements (11) enthält
und die Vakuumdüse (2) mit einem in das Verbindungselement (11) eingebetteten Basisende ausgeführt ist;
**dadurch gekennzeichnet, dass** der Magnet ein Neodym-Magnet (60) ist, und dass die Elektronenstrahl-Bestrahlungsvorrichtung vom Düsentyp ferner eine Kühlvorrichtung (8) umfasst, die die Vakuumdüse (2) kühlt, wobei die Kühlvorrichtung (8), während sie die Vakuumdüse (2) kühlt, den Neodym-Magneten (60) kühlt, um eine irreversible Entmagnetisierung zu verhindern,
wobei die Kühlvorrichtung (8) einen Kühlmittelkanal (83) umfasst, der ein Durchströmen eines Kühlmittels ermöglicht,
und der Kühlmittelkanal (83) zwischen der Vakuumdüse (2) und dem Neodym-Magneten (60) so angeordnet ist, dass er die Vakuumdüse (2) in dem Verbindungselement (11) umgibt.

## Revendications

1. Dispositif d'irradiation par faisceau d'électrons de type buse (1) comprenant une buse d'aspiration (2) pouvant être insérée dans une ouverture (m) d'un objet d'irradiation (O), l'ouverture (m) étant formée sur l'objet d'irradiation (O),
le dispositif d'irradiation par faisceau d'électrons de type buse (1) comprenant une chambre à vide (3) et un générateur de faisceau d'électrons (4) disposé dans la chambre à vide (3),
la buse d'aspiration (2) étant reliée à la chambre à vide (3) de manière à guider un faisceau d'électrons (E) provenant du générateur de faisceau d'électrons (4) et à émettre le faisceau d'électrons (E) vers l'extérieur,
le dispositif d'irradiation par faisceau d'électrons de type buse (1) comprenant :
un aimant qui entoure la buse d'aspiration (2) de l'extérieur et concentre le faisceau d'électrons (E) guidé dans la buse d'aspiration (2) ;
un mécanisme d'insertion/de retrait (7) qui insère et retire la buse d'aspiration (2) dans/de l'ouverture (m) de l'objet d'irradiation (O) ;
un élément de connexion (11) reliant la chambre à vide (3) et la buse d'aspiration (2),
l'élément de connexion (11) contenant l'aimant (60) à l'intérieur de l'élément de connexion (11),
la buse d'aspiration (2) étant disposée avec une extrémité de base encastrée dans l'élément de connexion (11) ;
**caractérisé en ce que** l'aimant est un aimant au néodyme (60), et **en ce que** le dispositif d'irradiation par faisceau d'électrons de type buse comprend en outre un mécanisme de refroidissement (8) qui refroidit la buse d'aspiration (2), le mécanisme de refroidissement (8) refroidissant l'aimant au néodyme (60) de manière à empêcher une démagnétisation irréversible pendant le refroidissement de la buse d'aspiration (2),
le mécanisme de refroidissement (8) comprenant un passage (83) de fluide de refroidissement qui permet le passage d'un fluide de refroidissement,
le passage (83) de fluide de refroidissement étant disposé entre la buse d'aspiration (2) et l'aimant au néodyme (60) de manière à entourer la buse d'aspiration (2) dans l'élément de connexion (11).
